# EUROPEAN PATENT APPLICATION

(11) **EP 4 759 232 A1**
(43) Date of publication of application: **17.06.2026**
(21) Application number: 24219079.1
(22) Date of filing: 11.12.2024
(51) Int. Cl.: A61B 5/16, A61B 5/18, A61B 5/00, G06V 40/16, G10L 25/63

(54) **A COMPUTER-IMPLEMENTED METHOD FOR DETERMINING A HUMAN'S INTERNAL EMOTIONS**

(71) Applicant: TOYOTA JIDOSHA KABUSHIKI KAISHA, Aichi-ken, 471-8571 (JP)
(72) Inventor: ABDELKAWY, Hazem, 1140 BRUSSELS (BE); DE WESER, Marleen, 1140 BRUSSELS (BE); GEBHARD, Patrick, 66123 SAARBRÜCKEN (DE); SCHNEEBERGER, Tanja, 66123 SAARBRÜCKEN (DE); HLADKÝ, Mirella, 66123 SAARBRÜCKEN (DE); BAUR, Tobias, 86159 AUGSBURG (DE); ANDRÉ, Elisabeth, 86159 AUGSBURG (DE)
(74) Representative: Cabinet Beau de Loménie

(57) **Abstract**

A computer-implemented method (20) for determining a human's internal emotions within its surrounding environment, comprising:
- collecting (21) a first set of data including behavioral cues of the human, and a second set of data including the surrounding environment of the human; and
- processing (22) said first and second sets of data to estimate a first internal emotional state of the human.

## Description

### BACKGROUND OF THE INVENTION

### 1. Field of the invention

The present disclosure relates to the field of emotion research and, more particularly, to an apparatus and a method for determining a human's internal emotions within their surrounding environment.

### 2. Description of Related Art

Increased traffic density on the roads necessitates that the vehicle drivers maintain continuous and undivided attention to the surrounding vehicles and obstacles on their travel path. However, the human capacity for sustained concentration is inherently limited, and as a result, drivers may experience lapses in attention that lead to critical driving errors. These errors can have severe consequences, including damage to the vehicle, injury to the driver, or harm to pedestrians and other vehicles in the vicinity.

Statistically, human error is implicated in approximately 94% of road accidents, with factors such as driver inattention, internal and external distractions, and insufficient situational awareness being the primary contributors. Consequently, human error represents a substantial risk to public safety on the roads.

To mitigate the impact of human error in driving, the development of Advanced Driver Assistance Systems (ADAS) has become a significant focus in automotive safety engineering. These systems are designed to automate, enhance, or adapt various driving tasks, thereby reducing the cognitive load on the driver and minimizing the risk of error.

ADAS typically employs a combination of sensors, including front-facing cameras, LiDAR, radar, and GPS, to monitor the vehicle's external environment. These sensors gather data related to the presence and characteristics of obstacles, which is then processed by machine learning classification models. The models analyse the captured data, assigning each detected object to a predefined training class - such as "pedestrians", "vehicles", "traffic signs", "animals" or "debris" - and determine the appropriate response, whether it be an autonomous vehicle action like automatic braking or an alert to the driver via visual, audio, or haptic feedback.

However, while these systems excel in detecting and responding to physical obstacles in the vehicle's environment, they often overlook a critical internal factor that can significantly influence driving safety: the emotional state of the driver. Emotions experienced during driving - such as anger, anxiety, surprise, or happiness - have been shown to substantially affect driving behaviour and decision-making processes. Negative emotions, in particular, can impair the driver's cognitive resources, leading to diverted attention, misinterpretation of the events, and delayed reaction times. These emotional influences can exacerbate the risks already posed by external distractions, making it imperative that ADAS not only consider environmental factors but also the driver's internal emotional state.

Current approaches to emotion recognition in driving contexts primarily rely on analysis of observable signals, such as facial expressions, voice tone, or physiological indicators, to infer the driver's emotional state. These methods often reference Ekman's basic emotion model, which maps facial expressions to specific emotions. However, this approach has significant limitations, as it does not account for the complexity of human emotions, which may not always manifest externally. For instance, negative emotions are frequently suppressed or regulated by individuals to maintain self-control, which means that these emotions may not be visibly apparent to an observer - or to a system relying solely on external cues. Additionally, emotions may be unconsciously experienced, further complicating their detection through observable signals alone.

In the context of ADAS, the failure to accurately recognize and respond to the driver's internal emotional state can result in inappropriate or ineffective system interventions. For example, a system that only recognizes visible anger might initiate calming measures when, in fact, the driver could be experiencing suppressed anxiety that requires a different response. This mismatch between the driver's true emotional state and the system's interpretation can undermine the effectiveness of ADAS, potentially increasing rather than decreasing the risk of accidents.

To address this challenge in this context and in a more general context where determining the internal emotions of a human is necessary, there is a need for an advanced emotion recognition approach that goes beyond surface-level indicators and incorporates psychological and emotional expertise. Such an approach would involve developing a more comprehensive model of emotion that considers both observable expressions and the internal, often hidden, aspects of emotional experience.

### SUMMARY

The object of the present invention is to at least substantially address the aforementioned drawbacks. In this respect, the aim of the invention is to provide a computer-implemented method for determining a human's internal emotions within their surrounding environment, comprising:
- collecting a first set of data including behavioral cues of the human, and a second set of data including the surrounding environment of the human; and
- processing said first and second sets of data to estimate a first internal emotional state of the human.

The invention addresses the challenges of accurately determining a person's internal emotional state by leveraging a computer-implemented method. This method is designed to capture and analyze both behavioral and environmental data to estimate the emotions a person, for example a driver, may be experiencing within their specific surroundings.

The method begins with the collection of two distinct sets of data. The first set of data focuses on behavioral cues exhibited by the person. These cues may include observable physical indicators such as facial expressions, body movements, gestures, and even vocal features like tone, pitch, or rhythm. These behavioral signals are crucial as they often provide direct, albeit external, signs of a person's emotional state.

The second set of data pertains to the surrounding environment of the person as the emotional state is often shaped not just by their internal responses but also by external conditions and stimuli. This includes various contextual elements that could influence or reflect the person's emotional state, such as social factors (the presence of other people, ongoing activities, and interactions).

Once the behavioral and environmental data are collected, the method involves processing these two sets of data to estimate the person's internal emotional state. Internal emotions are the true feelings experienced by the individual (the person), while observable expressions are the external behaviors that may or may not accurately reflect these internal states. The method thus ensures that the emotional state estimation is not solely based on outward appearances.

To this end, the processing step may comprise the use of algorithms that can interpret the behavioral cues in the context of the environmental data. For instance, a person's nervous behavior might be interpreted differently if they are in a high-pressure meeting versus a casual social gathering. More particularly, such algorithms predict the potential internal emotional state of the human based on these given input features, considering psychological and emotional theories that guide its structure and predictions. The algorithm may generate as an output a set of possible emotion classes. Of course, a person skilled in the art is undoubtedly capable of training such algorithm. They can effectively select relevant input features, defining appropriate emotion classes in accordance with the prior psychological and emotional knowledge.

Finally, the interpretation of these data sets allows the system to consider both what the person is doing and the context in which they are doing it. This dual consideration enables the system to make a more nuanced and accurate estimation of the person's emotions. Instead of relying solely on one type of data, which could lead to misinterpretations, the combination provides a richer, more comprehensive view of the emotional state. Of course, it is not necessary for the invention to determine the person's internal emotional state exclusively; it may also identify emotions in a more general sense, whether they are observable, internal, or unconscious.

Optionally, the computer-implemented method comprises modulating the internal emotions of the human towards a second internal emotional state based on the first internal emotional state of the human.

This optional step introduces the capability to not only assess but also modulate the internal emotions of the individual. After determining the first internal emotional state based on the collected data, the method can influence or adjust these emotions toward a second, desired emotional state. This modulation could be achieved through various means, such as altering environmental conditions or providing feedback to the individual, thereby helping to manage or steer their emotions in a specific direction.

Optionally, processing said first and second sets of data comprises modeling theoretical emotion knowledge representation based on the collected data, by using a Bayesian network.

The theoretical Emotion knowledge representation relates to a structured representation of theoretical emotion knowledge. This knowledge could be based on established psychological and emotional theories which categorizes emotions along dimensions of valence (positive vs. negative) and arousal (high vs. low energy), or other models that detail the relationship between emotions, physiological responses, and behavioral expressions.

This integration step is context-sensitive, meaning that the method takes into account the specific environment in which the person is situated -in this case, a driving environment. The driving environment is a controlled yet dynamic context where the individual's emotions may be influenced by various factors such as traffic conditions, interactions with other drivers, and the immediate physical surroundings. These factors can modulate both the internal emotions and the way they are expressed outwardly.

The method aims to modulate internal emotions based on observable expressions (within the collected data) by using a combination of the theoretical emotion knowledge representation and observable expressions, specifically tailored to the driving context (or another context as a surrounding environment). This differentiation is advantageous because internal emotions, which are often intrinsically regulated and influenced by sociocultural norms, do not always manifest through observable facial or behavioral expressions.

To model this complexity, a Bayesian network is employed. This Bayesian network integrates theoretical knowledge of emotions with observable data, enabling the modeling of probable relationships between internal emotional states and external expressions. This approach combines methods guided by psychological and emotional theories with data-driven approaches, offering a more comprehensive understanding of emotional states in driving context which is solely an example of context.

The Bayesian network thus captures the dynamic interplay between internal emotions and their potential expressions. In particular, it is advantageous to capture not only the visible expressions of emotion but also the underlying psychological and physiological processes. For instance, the method may consider how contextual factors, such as traffic conditions or the presence of other road users, can influence both the internal emotional state and its external manifestation.

Optionally, the surrounding environment is an environment where said human interacts with at least one individual, or is an environment where said human interacts with at least one object.

Of course, the terms 'human' and 'individual' are differentiated here for clarity. The 'human' refers to the person whose (internal) emotions are being determined, while the 'individual' denotes the person with whom the human interacts. Although both are human, this distinction is made here to clearly differentiate between the subject whose emotional state is assessed and the other party involved in the interaction.

Also, in this context, 'object' refers to a non-human entity with which the human may interact within the surrounding environment. Specifically, the term 'object' can include any physical or digital entity, such as a machine, device, or system. This distinction is important to clarify that the human's interactions are not limited to other individuals but may also involve interactions with objects, which could be, for instance, automated systems or other types of machinery.

Finally, the term "environment" refers here to the setting or space in which the human interacts, which can include a variety of different locations or contexts. For example, the environment could be a vehicle, a smart home, an office, a public space, or any other area. In other words, the term "environment' encompasses any space where the human may engage with other individuals or objects, including both physical and digital interactions.

The method may thus be employed in dynamic environments where human emotions may be influenced by social interactions or interactions with technology.

Optionally, the behavioral cues include facial features, and/or vocal features, and/or body language features, and/or interactional features.

This diverse range of cues allows the method to capture a comprehensive set of indicators that contribute to understanding the person's emotional state.

Optionally, the first set of data includes subjective emotional experience data associated with said human.

Optionally, the subjective emotional experience data includes said human-modeled emotional valence and its arousal levels.

The present disclosure further relates to a computer program set including instructions for executing the steps of the above-described computer-implemented method when said program set is executed by at least one computer.

This program set can use any programming language and take the form of source code, object code, or a code intermediate between source code and object code, such as a partially compiled form or any other desirable form.

The present disclosure further relates to a recording medium readable by at least one computer and having recorded thereon at least one computer program including instructions for executing the steps of the above-described computer-implemented method.

The present disclosure further relates to an apparatus for determining a human's internal emotions within their surrounding environment, comprising:
- a collecting module configured to collect a first set of data including behavioral cues of the human, and configured to collect a second set of data including the surrounding environment of the human; and
- a processing module configured to process said first and second sets of data to estimate a first internal emotional state of the human.

The apparatus referred to hereinafter as the determining apparatus, may be configured to carry out the above-mentioned method (referred to hereinafter as the determining method) and may have part or all of the above-described features. The determining apparatus may have the hardware structure of a computer.

Optionally, the apparatus comprises a modulating module configured to modulate the internal emotions of the human towards a second internal emotional state based on the first internal emotional state of the human.

Optionally, the collecting module comprises automated recognition sub-modules comprising audio, and/or physiological, and/or video sensing systems.

The audio sensing system focuses on analyzing vocal cues. For instance, it can assess changes in tone, pitch, and speech patterns to determine whether a person is feeling stressed, calm, or excited.

Another sub-module is the physiological sensing system, which monitors bodily responses to infer emotional states. This system might include sensors that track heart rate, skin conductance, or even brain activity through EEG (electroencephalography). Such physiological data provide a direct link to the body's natural responses to emotions, making this sub-system highly effective in stress management.

The video sensing system adds another layer of emotional recognition by analyzing facial expressions and body language through camera input. This sub-system utilizes computer vision techniques to detect subtle changes in facial muscles, eye movement, or posture for example.

The present disclosure further relates to a vehicle comprising an apparatus as described above.

The term "vehicle" may be directed to an apparatus capable of transporting people from one location to another, such as a car or a motorcycle.

### BRIEF DESCRIPTION OF THE DRAWINGS

Features, advantages, and technical and industrial significance of exemplary embodiments of the invention will be described below with reference to the accompanying drawings, in which like signs denote like elements, and wherein:
- Fig. 1 is a block diagram of a determining apparatus according to an embodiment of the present disclosure; and
- Fig. 2 is a flowchart of a determining method according to an embodiment of the present disclosure.

### DETAILED DESCRIPTION OF EMBODIMENTS

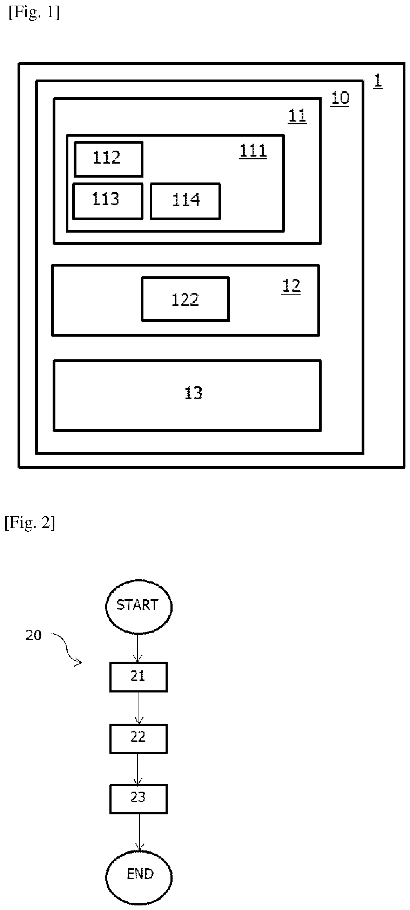

Figure 1 shows a block diagram of a determining apparatus 10 comprising a collecting module 11, a processing module 12, and a modulating module 13. The determining apparatus 10 may be located inside a vehicle 1 which may be, without limitation, an autonomous or semi-autonomous vehicle.

Alternatively, the determining apparatus 10 may be partially located outside the vehicle 1. As a first example, functions of the processing module 12 or the modulating module 13, are executed via cloud computing. As a second example, only functions of the processing module 12 is executed via cloud computing. Accordingly, the functions of the collecting module 11 and the modulating module 13 are then executed inside the vehicle 1.

The determining module 10 may be configured to determine a human's internal emotions within their surrounding environment. Accordingly, the determining apparatus 10 is designed to capture and analyse both human behavioural data and their environment to estimate their emotions.

Such vehicle 1 may be directed to an apparatus capable of transporting people or objects from one location to another, such as a car or a motorcycle, and may be categorized into different levels based on the extent of automation and human involvement in the driving tasks.

The determining apparatus 10 may comprise an electronic circuit, a processor (shared, dedicated, or group), a combinational logic circuit, a memory that executes one or more software programs, and/or other suitable components that provide the described functionality. In other words, the determining apparatus 10 may be a computer device. The determining apparatus 10 may be connected to a memory, which may store data, e.g., at least one computer program, which, when executed, carries out the determining method according to the present disclosure.

For example, the memory can be a ROM (for "Read Only Memory"), a CD ROM, a microelectronic circuit ROM, or in the form of magnetic storage means, for example, a diskette (floppy disk), a flash disk, a SSD (for "Solid State Drive") or a hard disk.

Alternatively, the memory can be an integrated circuit in which the program is incorporated, the circuit being adapted to execute the determining method or to be used in its execution.

The collecting module 11 is a module configured to collect a first set of data including behavioural cues of the human, and is a module configured to collect a second set of data including the surrounding environment of the human.

Accordingly, the collecting module 11 collects two distinct sets of data. The first set of data focuses on behavioral cues exhibited by the person. These cues may include observable physical indicators such as facial expressions, body movements, gestures, and even vocal features like tone, pitch, or rhythm. These behavioral signals are crucial as they often provide direct, albeit external, signs of a person's emotional state.

The first set of data may also include subjective emotional experience data associated with said human such as human-reported or human detected/modeled emotional valence (positive vs. negative) and its arousal levels (high vs. low energy).

The second set of data pertains to the surrounding environment of the person as the emotional state is often shaped not just by their internal responses but also by external conditions and stimuli. This includes various contextual elements that could influence or reflect the person's emotional state, such as social factors (the presence of other people, ongoing activities, and interactions).

To collect the first set of data and the second set of data, the collecting module 11 comprises automated recognition sub-modules 111 comprising for example an audio sensing system 112, and/or a physiological sensing system 113, and/or a video sensing system 114.

As explained above, the audio sensing system 112 focuses on analyzing vocal cues. For instance, it can assess changes in tone, pitch, and speech patterns to determine whether a person is feeling stressed, calm, or excited.

The physiological sensing system 113 monitors bodily responses to infer emotional states. This system might include sensors that track heart rate, skin conductance, or even brain activity through EEG (electroencephalography). Such physiological data provide a direct link to the body's natural responses to emotions, making this sub-system highly effective in stress management.

The video sensing system 114 adds another layer of emotional recognition by analyzing facial expressions and body language through camera input. This sub-system utilizes computer vision techniques to detect subtle changes in facial muscles, eye movement, or posture for example.

The collecting module 11 and its sub-modules recognition sub-modules may be implemented partially or fully as a hardware element of the determining apparatus 10.

The processing module 12 is a module configured to process said first and second sets of data to estimate a first internal emotional state of the human, wherein the internal emotions are the true feelings experienced by the individual (the person), while observable expressions are the external behaviors that may or may not accurately reflect these internal states.

The processing module 12 is also configured to model theoretical emotion knowledge representation based on the collected data, by using a Bayesian network.

The theoretical emotion knowledge representation relates to a structured representation of theoretical emotion knowledge. This knowledge could be based on established psychological and/or emotional theories which categorizes emotions along dimensions of valence (positive vs. negative) and arousal (high vs. low energy), or other models that detail the relationship between emotions, physiological responses, and behavioral expressions.

To model this complexity (the theoretical emotion knowledge), the Bayesian network integrates theoretical knowledge of emotions with observable data, enabling the modeling of probable relationships between internal emotional states and external expressions. This approach combines methods guided by psychological and/or emotional theories with data-driven approaches, offering a more comprehensive understanding of emotional states in driving contexts.

The Bayesian network thus captures the dynamic interplay between internal emotions and their potential expressions. In particular, it is advantageous to capture not only the visible expressions of emotion but also the underlying psychological and physiological processes. For instance, the method may consider how contextual factors, such as traffic conditions or the presence of other road users, can influence both the internal emotional state and its external manifestation.

The processing module 12 may comprise a sub-module 122 configured to execute an algorithm that is trained to interpret the behavioural cues in the context of the environmental data. In particular, based on the collected data (first and second sets of data), such algorithm predicts the potential internal emotional state of the human considering psychological (or emotional) theories and more specifically by using the Bayesian network that guide its structure and predictions. Of course, a person skilled in the art is undoubtedly capable of training such algorithm. They can effectively select relevant input features, defining appropriate emotion classes in accordance with the prior psychological and emotional knowledge.

The processing module 12 may be implemented as software running on the determining apparatus 10 or may be implemented partially as a hardware element of the determining apparatus 10.

The modulating module 13 is a module configured to modulate the first internal emotions of the human towards a second internal emotional state based on the first internal emotional state of the human.

Accordingly, after determining the first internal emotional state based on the collected data, the modulating module 13 is configured to influence or adjust the emotions of the human toward a second, desired emotional state. To achieve this, the modulating module 13 may comprise various means configured to alter environmental conditions or to provide feedback to the human, thereby helping to manage or steer their emotions in a specific direction.

This adjustment helps to manage emotions in a way that could enhance well-being, improve performance, or ensure safety.

It should be noted that the modulating module 13 may be implemented as software running on the determining apparatus 10. The modulating module 13 may also be implemented partially as a hardware element of the determining apparatus 10.

The tasks carried out by the determining apparatus 10 are detailed hereinafter with respect to the corresponding determining method, an embodiment of which is illustrated in Fig. 2.

Figure 2 is a flowchart of the determining method 20 according to an embodiment of the present disclosure. The determining method 20 is a method for determining the human's internal emotions within their surrounding environment.

The determining method 20 comprises a step 21 of collecting the first set of data including behavioral cues of the human, and the second set of data including the surrounding environment of the human. Such step 21 is performed by the collecting module 11.

As explained above, the behavioral cues include facial features, and/or vocal features, and/or body language features, and/or interactional features. The first set of data may also include subjective emotional experience data associated with said human such as human detected or modeled emotional valence and its arousal levels.

That is, the determining method 20 further comprises a step 22 of processing said first and second sets of data to estimate a first internal emotional state of the human. The step 22 is performed by the processing module 12.

The step 22 comprises modeling theoretical emotion knowledge based on the collected data, by using a Bayesian network.

Finally, the determining method 20 comprises an optional step 23 performed by the modulating module 13. The optional step 23 is performed to modulate the internal emotions of the human towards a second internal emotional state based on the first internal emotional state of the human.

Although the present disclosure refers to specific exemplary embodiments, modifications may be provided to these examples without departing from the general scope of the invention as defined by the claims. In particular, individual characteristics of the different illustrated/mentioned embodiments may be combined in additional embodiments. Therefore, the description and the drawings should be considered in an illustrative rather than in a restrictive sense. Of course, the invention is not limited to the field of driving assistance systems but can be used in any field where determination of a human's internal emotions within their surrounding environment is required.

## Claims

1. A computer-implemented method (20) for determining a human's internal emotions within its surrounding environment, comprising:
- collecting (21) a first set of data including behavioral cues of the human, and a second set of data including the surrounding environment of the human; and
- processing (22) said first and second sets of data to estimate a first internal emotional state of the human.

2. The computer-implemented method (20) according to claim 1, comprising modulating the internal emotions of the human towards a second internal emotional state based on the first internal emotional state of the human.

3. The computer-implemented method (20) according to claim 1 or 2, wherein processing said first and second sets of data comprises modeling theoretical emotion knowledge representation based on the collected data, by using a Bayesian network.

4. The computer-implemented method (20) according to any one of claims 1 to 3, wherein the surrounding environment is an environment where said human interacts with at least one individual, or is an environment where said human interacts with at least one object.

5. The computer-implemented method (20) according to any one of claims 1 to 4, wherein the behavioral cues include facial features, and/or vocal features, and/or body language features, and/or interactional features.

6. The computer-implemented method (20) according to any one of claims 1 to 5, wherein the first set of data includes subjective emotional experience data associated with said human.

7. The computer-implemented method (20) according to claim 6, wherein the subjective emotional experience data includes said human-modeled emotional valence and its arousal levels.

8. A computer program set including instructions for executing the steps of the computer-implemented method (20) of any one of claims 1 to 7 when said program set is executed by at least one computer.

9. A recording medium readable by at least one computer and having recorded thereon at least one computer program including instructions for executing the steps of the computer-implemented method (20) of any one of claims 1 to 7.

10. An apparatus (10) for determining a human's internal emotions within its surrounding environment, comprising:
- a collecting module (11) configured to collect a first set of data including behavioral cues of the human, and configured to collect a second set of data including the surrounding environment of the human; and
- a processing module (12) configured to process said first and second sets of data to estimate a first internal emotional state of the human.

11. The apparatus (10) according to claim 10, comprising a modulating module (13) configured to modulate the internal emotions of the human towards a second internal emotional state based on the first internal emotional state of the human.

12. The apparatus (10) according to claim 10 or 11, wherein the collecting module (11) comprises automated recognition sub-modules (111) comprising audio (112), and/or physiological (113), and/or video sensing systems (114).

13. A vehicle (1) comprising an apparatus (10) according to any one of claims 10 to 12.

## Amended claims

### Amended claims in accordance with Rule 137(2) EPC.

1. A computer-implemented method (20) for determining a human's internal emotions within its surrounding environment, the surrounding environment being an environment where said human interacts with at least one object, the computer-implemented method (20) comprising:
- collecting (21) a first set of data including behavioral cues of the human, and a second set of data including the surrounding environment of the human; and
- processing (22) said first and second sets of data to estimate a first internal emotional state of the human,
wherein the computer-implemented method is **characterized in that** it comprises:
- modulating the internal emotions of the human towards a second internal emotional state based on the first internal emotional state of the human by altering the environmental conditions, the second internal emotional state being a desired emotional state.

2. The computer-implemented method (20) according to claim 1, wherein processing said first and second sets of data comprises modeling theoretical emotion knowledge representation based on the collected data, by using a Bayesian network.

3. The computer-implemented method (20) according to claim 1 or 2, wherein the surrounding environment is an environment where said human interacts with at least one individual.

4. The computer-implemented method (20) according to any one of claims 1 to 3, wherein the behavioral cues include facial features, and/or vocal features, and/or body language features, and/or interactional features.

5. The computer-implemented method (20) according to any one of claims 1 to 4, wherein the first set of data includes subjective emotional experience data associated with said human.

6. The computer-implemented method (20) according to claim 5, wherein the subjective emotional experience data includes said human-modeled emotional valence and its arousal levels.

7. A computer program set including instructions for executing the steps of the computer-implemented method (20) of any one of claims 1 to 6 when said program set is executed by at least one computer.

8. A recording medium readable by at least one computer and having recorded thereon at least one computer program including instructions for executing the steps of the computer-implemented method (20) of any one of claims 1 to 6.

9. An apparatus (10) for determining a human's internal emotions within its surrounding environment, the surrounding environment being an environment where said human interacts with at least one object, the apparatus (10) comprising:
- a collecting module (11) configured to collect a first set of data including behavioral cues of the human, and configured to collect a second set of data including the surrounding environment of the human; and
- a processing module (12) configured to process said first and second sets of data to estimate a first internal emotional state of the human,
wherein the apparatus (10) is **characterized in that** it comprises:
- a modulating module (13) configured to modulate the internal emotions of the human towards a second internal emotional state based on the first internal emotional state of the human by altering the environmental conditions, the second internal emotional state being a desired emotional state.

10. The apparatus (10) according to claim 9, wherein the collecting module (11) comprises automated recognition sub-modules (111) comprising audio (112), and/or physiological (113), and/or video sensing systems (114).

11. A vehicle (1) comprising an apparatus (10) according to claim 9 or 10.
